# EUROPÄISCHE PATENTSCHRIFT

(11) **EP 0 636 359 B1**
(45) Veröffentlichungstag und Bekanntmachung des Hinweises auf die Patenterteilung: **17.09.1997**
(21) Anmeldenummer: 94110443.2
(22) Anmeldetag: 05.07.1994
(51) Int. Cl.: A61K 7/32, A61K 7/48

(54) **Desodorierende Wirkstoffkombinationen auf der Basis von alpha-omega-Alkandicarbonsäuren und Wollwachssäuren**
Deodorant mixture based on alpha-omega alkanoic dicarboxylic acids and wool wax acids
Combination d'agents déodorants à base d'acides alcaniques alpha-omega dicarboxyliques et des acides de laine

(30) Priorität: 20.07.1993 DE 4324219
(43) Veröffentlichungstag der Anmeldung: 01.02.1995
(73) Patentinhaber: Beiersdorf Aktiengesellschaft, D-20245 Hamburg (DE)
(72) Erfinder: Klier, Manfred, Dr., D-20521 Aumühle (DE); Wolf, Florian, Dr., D-20251 Hamburg (DE); Eitrich, Anja, D-20255 Hamburg (DE)

(56) Entgegenhaltungen:
- EP-A- 0 036 134
- EP-A- 0 129 014
- LU-A- 73 343

## Beschreibung

Die vorliegende Erfindung betrifft kosmetische Wirkstoffkombinationen, insbesondere Wirkstoffkombinationen als wirksames Prinzip in kosmetischen Desodorantien.

Kosmetische Desodorantien dienen dazu, Körpergeruch zu beseitigen, der entsteht, wenn der an sich geruchlose frische Schweiß durch Mikroorganismen zersetzt wird. Den Üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, daß die gesamte Mikroflora der Haut beeinträchtigt werden kann.

Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht. Dies bemerkt schon Plautus (244 - 184 v.u.Z. ) in seiner "Gespenstergeschichte" ("Mostellaria", 1. Aufzug, 3.Auftritt: "ubi sese sudor cum unguentis consociavit, ilico itidem olent, quasi cum una multa iura confudit cocus. quid olant nescias, nisi id unum, ut male olere intellegas.")

Allerdings werden die meisten kosmetischen Desodorantien, wie die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Zubereitungen, insbesondere kosmetischer Desodorantien, ist allerdings nicht selten problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Bekannt und gebräuchlich sind sowohl flüssige Desodorantien, beispielsweise Aerosolsprays, Roll-ons und dergleichen als auch feste Zubereitungen, beispielsweise DeoStifte ("Sticks"), Puder, Pudersprays, Intimreinigungsmittel usw.

Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Noch eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Es wurde überraschenderweise gefunden, und darin liegt die Lösung all dieser Aufgaben, daß kosmetische Desodorantien, enthaltend Gemische aus
I) α,Ω-Alkandicarbonsäuren und
II) Wollwachssäuren oder Wollwachssäurekomponenten
in wirksamer Konzentration, den Nachteilen des Standes der Technik abhelfen.

Zwar beschreibt die Europäische Patentanmeldungsschrift EP-0 036 134 Desodorierende Zusammensetzungen, gekennzeichnet durch einen Gehalt an Derivaten mittel- bis längerkettiger Alkansäuren, welche auch die α,Ω-Alkandicarbonsäuren der allgemeinen Formel 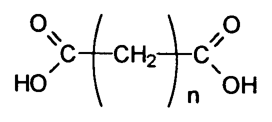 umfassen,
mit n = 4 bis 10, ein Hinweis auf die hiermit vorgelegte Lehre findet sich in dieser Schritt jedoch nicht.

Ferner beschreibt die Deutsche Offenlegungsschrift DE-OS 27 03 642 desodorierende Mittel für die Körperhygiene, welche unter anderem gewisse α,ω-Alkandicarbonsäuren umfaßt, ein Hinweis auf die hiermit vorgelegte Lehre findet sich jedoch auch in dieser Schritt nicht.

Aus dem Aufsatz "Antimicrobial Factors in Wool Wax" (Australian Journal of Chemistry, 1971, 24, Seiten 153 ff.) ist bekannt, daß in manchen Wollwachschargen antimikrobielle Faktoren enthalten sind. Ein Hinweis in Richtung der vorliegenden Erfindung findet sich am angegebenen Orte jedoch nicht.

Schließlich beschreibt die Europäische Patentanmeldung 129 014 desodorierende Mittel für die Körperhygiene auf der Grundlage von Wollwachssäuren, ein Hinweis auf die hiermit vorgelegte Lehre findet sich jedoch auch in dieser Schritt nicht.

Erfindungsgemäß werden die α,ω-Alkandicarbonsäuren bevorzugt gewährt aus der Gruppe der Substanzen, die von der generischen Formel 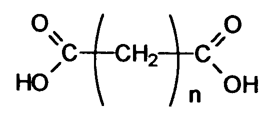 beschrieben werden,
wobei n Zahlen von 1 bis 8 annehmen kann.
- n = 1 :: Malonsäure
- n = 2 :: Bernsteinsäure
- n = 3 :: Glutarsäure
- n = 4 :: Adipinsäure
- n = 5 :: Pimelinsäure
- n = 6 :: Korksäure
- n = 7 :: Azelainsäure
- n = 8 :: Sebacinsäure

Als Wollwachs oder Wollfett wird der bei der Rohwollwäsche anfallende fett- bis wachsartige Bestandteil der Rohschafwolle bezeichnet. Das Wollwachs besteht aus aus einem Gemisch von Fettsäureestern höherer Alkohole und aus freien Fettsäuren.

Die Hauptbestandteile der Wollwachssäuren sind
(a) gesättigte unsubstituierte Carbonsäuren, gemäß der Formel

   CH₃-(CH₂)ₙCH₂-COOH,
(b) α-Hydroxycarbonsäuren, gemäß der Formel 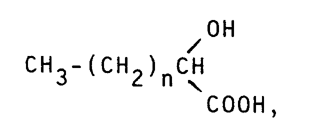
(c) Ω-Hydroxycarbonsäuren, gemäß der Formel

   HO-CH₂-(CH₂)ₙCH₂-COOH,
(d) Isocarbonsäuren, gemäß der Formel 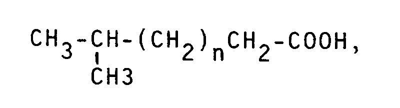
(e) α-Hydroxy-isocarbonsäuren, gemäß der Formel 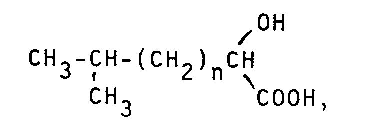
(f) Ω-Hydroxy-isocarbonsäuren, gemäß der Formel 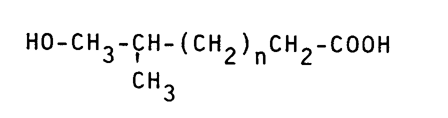
(g) Anteisocarbonsäuren, gemäß der Formel 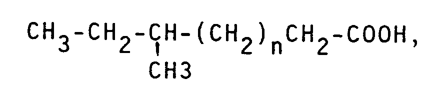
(h) α-Hydroxy-anteisocarbonsäuren, gemäß der Formel 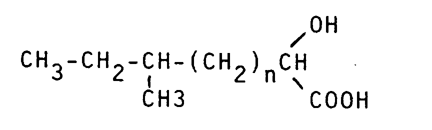
(i) Ω-Hydroxy-anteisocarbonsäuren, gemäß der Formel

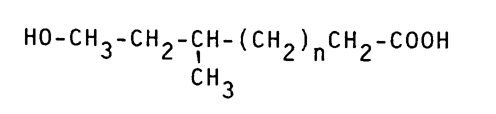

Dabei nimmt n gewöhnlich Werte von 7 - 31 an. Reprasentative Zusammensetzungen der Wollwachssäuren werden beispielsweise in "Parfümerie und Kosmetik", 59. Jahrgang, Nr.12/78, S.429, 430 sowie im "Lexikon der Hilfsstoffe für Pharmazie, Kosmetik und angrenzende Gebiete" von H.P.Fiedler, 1989, 3.Auflage, Editio Cantor Aulendorf, beschrieben.

Rohwollwachssäuren sind für kosmetische Zwecke nicht geeignet, statt ihrer werden für gewöhnlich destillierte Wollwachssäuren eingesetzt. Dieser Umstand und entsprechende Verfahren zur Raffinierung der Rohwollwachssäuren sind dem Fachmann bekannt.

Typischerweise bestehen Wollwachssäuren aus ca. 60 % gesättigten, unsubstituierten Carbonsäuren, bis ca. 30 % α-Hydroxycarbonsäuren und ca. 5 % Ω-Hydroxycarbonsäuren, wobei der Rest von ca. 5 % im wesentlichen von den anderen vorgenannten Carbonsäuretypen gebildet wird.

Insbesondere zeichnen sich die erfindungsgemäßen Wollwachssäuren vorteilhaft durch folgende kennzeichnende Parameter aus:

| | |
|---|---|
| Tropfpunkt | 50 - 54° C |
| Säurezahl | 166 - 170 |
| Verseifungszahl | 175 - 190 |
| OH - Zahl | 60 - 80 |
| Jodzahl | 7 - 12 |

Es wird angenommen, daß insbesondere die α-Hydroxycarbonsäuren einen wesentlichen Beitrag zur erfindungsgemäßen Wirkung leisten. Erfindungsgemäß sind daher auch kosmetische Desodorantien, enthaltend Gemische aus
I) α,Ω-Alkandicarbonsäuren und
II)
   - α-Hydroxycarbonsäuren, gemäß der Formel 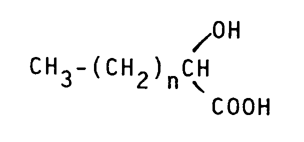 und/oder
   - α-Hydroxy-isocarbonsäuren, gemäß der Formel 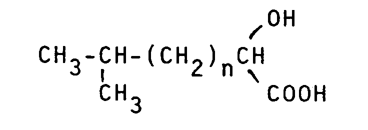 und/oder
   - α-Hydroxy-anteisocarbonsäuren, gemäß der Formel 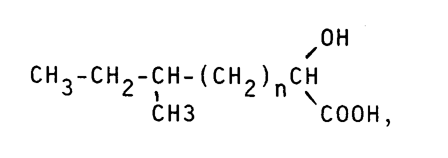
   wobei n jeweils eine Zahl von 7 bis 31 darstellt.

Besonders vorteilhaft im Sinne der vorliegenden Erfindung ist, α-Hydroxycarbonsäuren zu verwenden, welche C₁₆-Körper darstellen, die also am α-Kohlenstoffatom eine verzweigte oder unverzweigte C₁₄H₂₉-Kette tragen.

Vorteilhaft ist weiter, Wollwachssäuregemische zu verwenden, in welchen der Gehalt an α-Hydroxycarbonsäuren mindestens 20 - 30 Gew.-%, bezogen auf die Gesamtzusammensetzung beträgt.

Nach einer weiteren vorteilhaften Ausführungsform der vorliegenden Erfindung werden als Wollwachssäurekomponenten eine oder mehrere
(a) gesättigte unsubstituierte Carbonsäuren, gemäß der Formel

   CH₃-(CH₂)ₙCH₂-COOH,

   und/oder
(b) α-Hydroxycarbonsäuren, gemäß der Formel 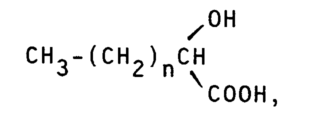 und/oder
(c) Ω-Hydroxycarbonsäuren, gemäß der Formel

   HO-CH₂-(CH₂)ₙCH₂-COOH,

   und/oder
(d) Isocarbonsäuren, gemäß der Formel 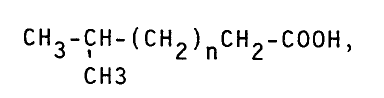 und/oder
(e) α-Hydroxy-isocarbonsäuren, gemäß der Formel 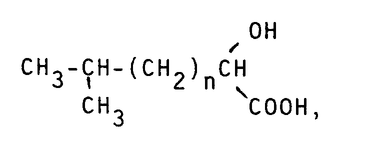 und/oder
(f) Ω-Hydroxy-isocarbonsäuren, gemäß der Formel 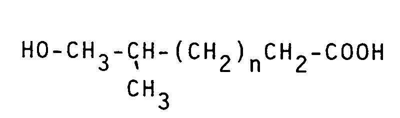 und/oder
(g) Anteisocarbonsäuren, gemäß der Formel 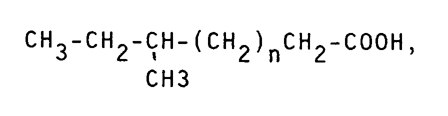 und/oder
(h) α-Hydroxy-anteisocarbonsäuren, gemäß der Formel 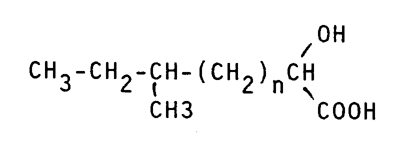 und/oder
(i) Ω-Hydroxy-anteisocarbonsäuren, gemäß der Formel 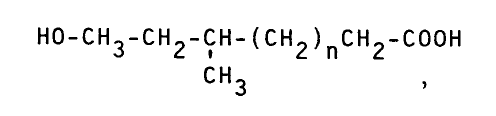
wobei n Werte von 7 - 31 annimmt, in Kombination mit anderen in Kosmetika üblichen Wirkstoffen (Ersatzwirkstoffe), Hilfs-, Verschnitt- und/oder Zusatzstoffen eingesetzt.

Vorteilhaft liegen dann die Verschnittstoffe und/oder Ersatzwirkstoffe in einer Konzentration bis zu 50 Gew.-Teilen vor, bevorzugt bis zu 35 Gew.-Teilen, bezogen auf 100 Gew.-Teile der Gesamtmenge, welche sich aus der Summe der Wollwachssäurekomponenten und diesen Ersatzwirkstoffen und/oder Verschnittstoffen zusammensetzt.

Als besonders vorteilhafte Verkörperung der vorliegenden Erfindung werden kosmetische Desodorantien mit einem Gehalt an
I) Adipinsäure
II) Wollwachssäuren bzw. Wollwachssäurekomponenten und
angesehen.

Vorteilhafte Verkörperungen der vorliegenden Erfindung stellen auch kosmetische Desodorantien mit einem Gehalt an
I) Azelainsäure
II)
   - α-Hydroxycarbonsäuren, gemäß der Formel 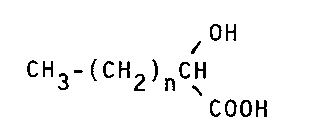 und/oder
   - α-Hydroxy-isocarbonsäuren, gemäß der Formel 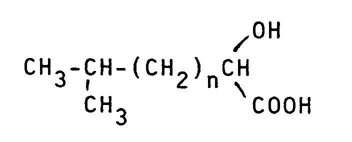 und/oder
   - α-Hydroxy-anteisocarbonsäuren, gemäß der Formel 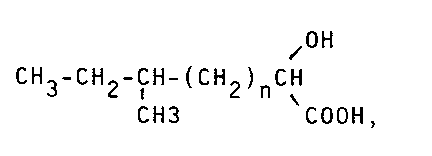
   wobei n jeweils eine Zahl von 7 bis 31 darstellt.

Es ist von Vorteil, den Gehalt an
I) α,Ω-Alkandicarbonsäuren und
II) Wollwachssäuren bzw. Wollwachssäurekomponenten
so zu wählen, daß Verhältnisse von I) und II) wie 5 : 1 bis 1 : 5, insbesondere wie etwa 1 : 1 entstehen.

Erfindungsgemäß ist ferner ein Verfahren zur Bekämpfung des durch mikrobielle Zersetzung apokrinen Schweißes hervorgerufenen menschlichen Körpergeruches, dadurch gekennzeichnet, daß eine wirksame Menge an

Gemischen aus
I) α,Ω-Alkandicarbonsäuren und
II) Wollwachssäuren bzw. Wollwachssäurekomponenten
ganz besonders
Gemischen aus
I) α,ω-Alkandicarbonsäuren und
II) α-Hydroxycarbonsäuren, gemäß der Formel 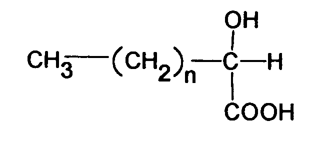 und/oder
α-Hydroxy-isocarbonsäuren, gemäß der Formel 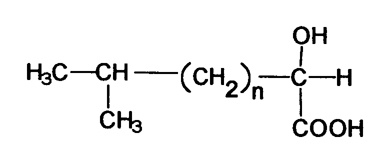 und/oder
α-Hydroxy-anteisocarbonsäuren, gemäß der Formel 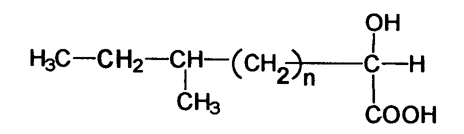 wobei n jeweils eine Zahl von 7 bis 31 darstellt,
welche gegebenenfalls in einem geeigneten kosmetischen Träger vorliegen können, auf die Haut aufgetragen wird.

Erfindungsgemäß ist schließlich auch die kosmetische Verwendung von Gemischen aus
I) α,ω-Alkandicarbonsäuren und
II) Wollwachssäuren bzw. Wollwachssäurekomponenten
ganz besonders Gemischen aus
I) α,ω-Alkandicarbonsäuren und
II) α-Hydroxycarbonsäuren, gemäß der Formel 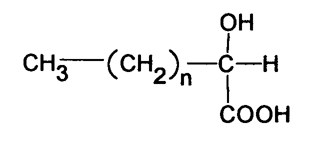 und/oder
α-Hydroxy-isocarbonsäuren, gemäß der Formel 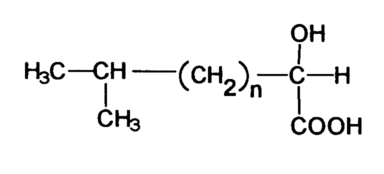 und/oder
α-Hydroxy-anteisocarbonsäuren, gemäß der Formel 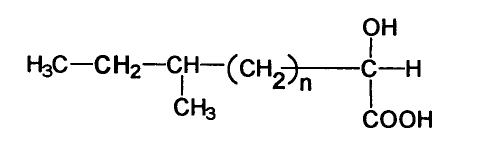 wobei n jeweils eine Zahl von 7 bis 31 darstellt,
welche gegebenenfalls in einem geeigneten kosmetischen Träger vorliegen können, zur Bekämpfung grampositiver, insbesondere coryneformer Bakterien, beziehungsweise die kosmetische Verwendung von Gemischen aus
I) α,ω-Alkandicarbonsäuren und
II) Wollwachssäuren bzw. Wollwachssäurekomponenten
ganz besonders Gemischen aus
I) α,ω-Alkandicarbonsäuren und
II) α-Hydroxycarbonsäuren, gemäß der Formel 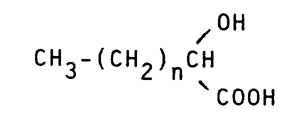 und/oder
   - α-Hydroxy-isocarbonsäuren, gemäß der Formel 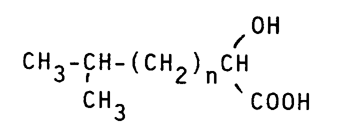 und/oder
   - α-Hydroxy-anteisocarbonsäuren, gemäß der Formel 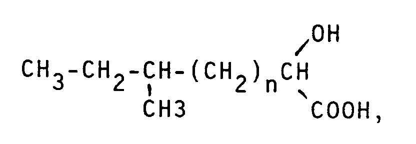
   wobei n jeweils eine Zahl von 7 bis 31 darstellt,
welche gegebenenfalls in einem geeigneten kosmetischen Träger vorliegen können, zur Bekämpfung des durch die mikrobielle Zersetzung des apokrinen Schweißes hervorgerufenen Körpergeruches.

Die erfindungsgemäßen kosmetischen Desodorantien sind besonders vorteilhaft dadurch gekennzeichnet, daß die Wollwachssäuren bzw. die Wollwachssäurekomponenten in Konzentrationen von 0,05 - 10,00 Gew.-%, bevorzugt 0,1 - 5,0 Gew.-%, vorliegen, jeweils bezogen auf das Gesamtgewicht der Zubereitungen.

Die erfindungsgemäßen kosmetischen Desodorantien sind besonders vorteilhaft dadurch gekennzeichnet, daß die α,Ω-Alkandicarbonsäure oder die α,Ω-Alkandicarbonsäuren in Konzentrationen von 0,01 - 10,00 Gew.-%, bevorzugt 0,05 - 5,0 Gew.-%, vorliegen, jeweils bezogen auf das Gesamtgewicht der Zubereitungen.

Die erfindungsgemäßen kosmetischen Desodorantien können in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, als DeoStifte (Deo-Sticks) und in Form von aus normalen Flaschen und Behältern auftragbaren W/O- oder O/W-Emulsionen, z.B. Crèmes oder Lotionen. Weiterhin können die kosmetischen Desodorantien vorteilhaft in Form von desodorierenden Tinkturen, desodorierenden Intimreinigungsmitteln, desodorierenden Shampoos, desodorierenden Dusch- oder Badezubereitungen, desodorierenden Pudern oder desodorierenden Pudersprays vorliegen.

Als übliche kosmetische Trägerstoffe zur Herstellung der erfindungsgemäßen desodorierenden Zubereitungen können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyvinylpyrrolidon, daneben aber auch in kleinen Mengen cyclische Silikonöle (Polydimethylsiloxane) sowie flüssige Polymethylphenylsiloxane niedriger Viskosiät.

Als Treibmittel für erfindungsgemäße, aus Aerosolbehältern versprühbare kosmetische Desodorantien sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Als Emulgatoren zur Herstellung der erfindungsgemäßen kosmetischen Desodorantien, welche vorteilhaft als flüssige Zubereitungen mittels einer Roll-on-Vorrichtung auf die gewünschten Hautbereiche aufgetragen werden sollen, und die in den Zubereitungen in geringer Menge, z.B. 2 bis 5 Gewichts.-%, bezogen auf die Gesamtzusammensetzung, verwendet werden können, haben sich nichtionogene Typen, wie Polyoxyethylen-Fettalkoholether, z.B. Cetostearylalkoholpolyethylenglykolether mit 12 bzw. 20 angelagerten Ethylenoxid-Einheiten pro Molekül, Cetostearylalkohol sowie Sorbitanester und Sorbitanester-Ethylenoxid-Verbindungen (z.B. Sorbitanmonostearat und Polyoxyethylensorbitanmonostearat) und langkettige höhermolekulare wachsartige Polyglykolether als geeignet erwiesen.

Zusätzlich zu den genannten Bestandteilen können den erfindungsgemäßen desodorierenden kosmetischen Zubereitungen, deren pH-Wert vorzugsweise z.B. durch übliche Puffergemische auf 4,0 bis 7,0 insbesondere 5,0 bis 6,5, eingestellt wird, Parfüm, Farbstoffe, Antioxidantien (z.B. α-Tocopherol und seine Derivate oder Butylhydroxytoluol (BHT = 2,6-Di-Tert.-butyl-4-methylphenol) in Mengen von 0,01 bis 0,03 %, bezogen auf die Gesamtzusammensetzung), Suspendiermittel, Puffergemische oder andere übliche kosmetische Grundstoffe beigemischt werden.

Der pH-Wert der erfindungsgemäßen kosmetischen Desodorantien wird bevorzugt so eingestellt, daß die erfindungsgemäßen Säurekomponenten im wesentlichen als Säuren, und nicht als Anionen, vorliegen, also bevorzugt im sauren bis neutralen Bereich, insbesondere im pH-Bereich von 5,0 - 6,5.

Die jeweils einzusetzenden Mengen an kosmetischen Trägerstoffen und Parfüm können in Abhängigkeit von der Art des jeweiligen Produktes vom Fachmann durch einfaches Ausprobieren leicht ermittelt werden.

Zur Parfümierung sind gegebenenfalls auch solche Substanzen und Parfümöle geeignet, die stabil sind, die Haut nicht reizen und bereits als solche antibakterielle oder bakteriostatische Eigenschaften besitzen.

Die Herstellung der kosmetischen Zubereitungen erfolgt abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat, gegebenenfalls unter Erwärmen hergestellt und dann emulgiert.

Ansonsten sind die üblichen Maßregeln für das Zusammenstellen von kosmetischen Formulierungen zu beachten, die dem Fachmann geläufig sind.

Sollen die erfindungsgemäßen Zusammensetzungen in Pudersprays eingearbeitet werden, so können die Suspensionsgrundlagen dafür vorteilhaft gewählt werden aus der Gruppe Kieselsäuregele (z.B. solche die unter dem Handelsnamen Aerosil^{R} erhältlich sind), Kieselgur, Talkum, modifizierte Stärke, Titandioxid, Seidenpulver, Nylonpulver, Polyethylenpulver und verwandten Stoffen.

Es folgen vorteilhafte Ausführungsbeispiele der vorliegenden Erfindung. Die angegebenen Zahlenwerte beziehen sich stets auf Gew.-%, sofern nicht ausdrücklich etwas Anderes vermerkt wird.

In den Beispielen bedeutet der Begriff "WWS" eine Wollwachssäurefraktion, welche gewonnen wurde aus Rohwollwachssäure durch Kurzwegdestillation im bei 10⁻¹ bar aus dem Destillationstemperaturintervall von 150 - 200° C. Der Anteil an α-Hydroxycarbonsäuren beträgt dabei ca. 22 - 27 %.

| Beispiel 1 - 4 | | | | |
|---|---|---|---|---|
| Pumpspray | 1 | 2 | 3 | 4 |
| Ethanol | 60,00 | 63,00 | 60,00 | 60,00 |
| Propylenglycol | 3,00 | 2,50 | 3,00 | 3,00 |
| PEG-40-hydriertes Rizinusöl | 2,50 | 2,50 | 2,50 | 2,00 |
| Adipinsäure | 0,45 | 0,30 | - | - |
| Azelainsäure | - | - | 0,35 | 0,30 |
| α-Hydroxypalmitinsäure | - | 0,25 | - | - |
| WWS | 0,30 | - | 0,35 | 0,35 |
| Parfum | q.s. | q.s. | q.s. | q.s. |
| Wasser, NaOH 10-%ig ad pH 5 | -------------ad 100,00------------- | | | |

| Beispiel 5 - 6 | | |
|---|---|---|
| Roll-on Gel | 5 | 6 |
| Ethanol | 50,00 | 45,00 |
| Hydroxyethylcellulose | 0,50 | 0,50 |
| Steareth-20 ("Brij 78^{R}") | 1,50 | 1,50 |
| Adipinsäure | - | 0,50 |
| Azelainsäure | 0,45 | - |
| WWS | 0,35 | 0,30 |
| Parfum | q.s. | q.s. |
| Wasser, NaOH 10-%ig ad pH 5 | -----ad 100,00------ | |

| Beispiel 7 - 8 | | |
|---|---|---|
| Roll-On Emulsion (O/W) | 7 | 8 |
| Steareth-10 ("Brij 76^{R}") | 4,00 | 4,00 |
| Cetylalkohol | 2,00 | 1,50 |
| Mineralöl DAB 9 | 7,00 | 7,00 |
| PPG-15 Stearylether | 4,50 | 4,50 |
| Methylparaben | 0,20 | 0,20 |
| Dipropylenglycol | 2,50 | 2,50 |
| Adipinsäure | 0,80 | - |
| Bernsteinsäure | - | 1,10 |
| WWS | 0,45 | 0,55 |
| Parfum | q.s. | q.s. |
| Wasser, NaOH 10-%ig ad pH 5 | -----ad 100,00------ | |

| Beispiel 9 - 10 | | |
|---|---|---|
| Wachsstift (Wasserfrei) | 9 | 10 |
| Trilaurin | 38,00 | 38,00 |
| Caprylic/capric Triglyceride ("Miglyol 812^{R}") | 29,50 | 29,50 |
| Glycerylstearat, selbstemulgierend | 8,50 | 8,50 |
| Bienenwachs | 21,00 | 21,00 |
| Adipinsäure | 0,50 | - |
| Azelainsäure | - | 0,60 |
| WWS | 0,80 | 0,60 |
| Parfum | q.s. | q.s. |
| Wasser, NaOH 10-%ig ad pH 5 | -----ad 100,00------ | |

## Patentansprüche

1. Kosmetische Desodorantien, enthaltend Gemische aus
I) α,ω-Alkandicarbonsäuren und
II) Wollwachssäuren oder Wollwachssäurekomponenten
in wirksamer Konzentration.

2. Kosmetische Desodorantien nach Anspruch 1, dadurch gekennzeichnet, daß die erfindungsgemäßen α,ω-Alkandicarbonsäuren gewählt werden aus der Gruppe der Substanzen, die von der generischen Formel 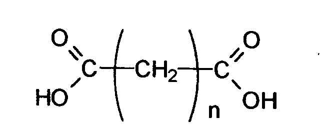 beschrieben werden, wobei n Zahlen von 1 bis 8 annehmen kann.

3. Kosmetische Desodorantien nach Anspruch 1, dadurch gekennzeichnet, daß die Wollwachssäurekomponenten gewährt werden aus der Gruppe der
(a) gesättigten unsubstituierten Carbonsäuren, gemäß der Formel
CH₃-(CH₂)ₙ-CH₂-COOH
(b) α-Hydroxycarbonsäuren, gemäß der Formel 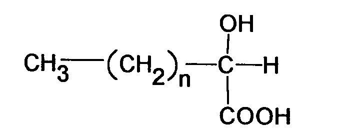
c) ω-Hydroxycarbonsäuren, gemäß der Formel
HO-CH₂-(CH₂)ₙ-CH₂-COOH
(d) Isocarbonsäuren, gemäß der Formel 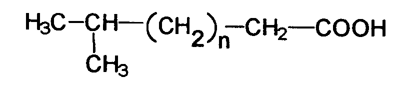
(e) α-Hydroxy-isocarbonsäuren, gemäß der Formel 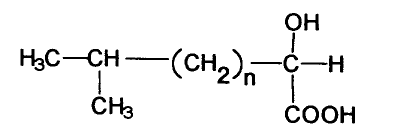
(f) ω-Hydroxy-isocarbonsäuren, gemäß der Formel 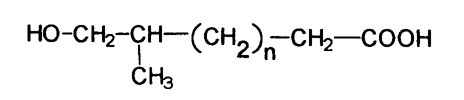
(g) Anteisocarbonsäuren, gemäß der Formel 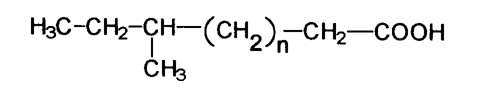
(h) α-Hydroxy-anteisocarbonsäuren, gemäß der Formel 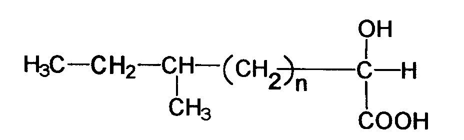
(i) ω-Hydroxy-anteisocarbonsäuren, gemäß der Formel 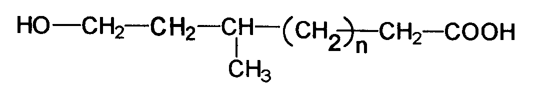
wobei jeweils n Werte von 7 - 31 annimmt.

4. Kosmetische Desodorantien nach Anspruch 1, dadurch gekennzeichnet, daß Wollwachssäuregemische verwendet werden, in welchen der Gehalt an α-Hydroxycarbonsäuren mindestens 20 - 30 Gew.-%, bezogen auf die Gesamtzusammensetzung beträgt.

5. Kosmetische Desodorantien nach Anspruch 1, dadurch gekennzeichnet, daß die Wollwachssäuren sich durch folgende kennzeichnende Parameter auszeichnen:
| | |
|---|---|
| Tropfpunkt | 50 - 54° C |
| Säurezahl | 166 - 170 |
| Verseifungszahl | 175 - 190 |
| OH-Zahl | 60 - 80 |
| Jodzahl | 7 - 12. |

6. Kosmetische Desodorantien nach Anspruch 1, dadurch gekennzeichnet, daß die Wollwachssäuren bzw. die Wollwachssäurekomponenten in Konzentrationen von 0,05 - 10,00 Gew.-%, bevorzugt 0,1 - 5,0 Gew.-% und die α,ω-Alkandicarbonsäure oder die α,ω-Alkandicarbonsäuren in Konzentrationen von 0,01 - 10,00 Gew.-%, bevorzugt 0,05 - 5,0 Gew.-%, vorliegen, jeweils bezogen auf das Gesamtgewicht der Zubereitungen.

7. Kosmetische Desodorantien nach Anspruch 1, dadurch gekennzeichnet, daß der Gehalt an
I) α,ω-Alkandicarbonsäuren und
II) Wollwachssäuren bzw. Wollwachssäurekomponenten
so gewährt wird, daß Verhältnisse von I) und II) wie 5 : 1 bis 1 : 5, insbesondere wie etwa 1 : 1 entstehen.

8. Kosmetische Verwendung von Gemischen aus
I) α,ω-Alkandicarbonsäuren und
II) Wollwachssäuren bzw. Wollwachssäurekomponenten,
welche gegebenfalls in einem geeigneten kosmetischen Träger vorliegen können, zur Bekämpfung grampositiver, insbesondere coryneformer Bakterien, zur Bekämpfung des durch die mikrobielle Zersetzung des apokrinen Schweißes hervorgerufenen Körpergeruches.

## Claims

1. Cosmetic deodorants comprising mixtures of
I) α,ω-alkanedicarboxylic acids and
II) wool wax acids or wool wax acid components
in effective concentration.

2. Cosmetic deodorants according to Claim 1, characterized in that the α,ω-alkanedicarboxylic acids according to the invention are chosen from the group of substances which are described by the generic formula 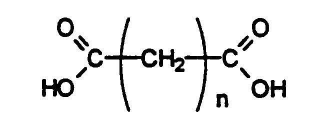 wherein n can assume numbers from 1 to 8.

3. Cosmetic deodorants according to Claim 1, characterized in that the wool wax acids are chosen from the group consisting of
(a) saturated unsubstituted carboxylic acids according to the formula
CH₃-(CH₂)ₙ-CH₂-COOH
(b) α-hydroxycarboxylic acids according to the formula 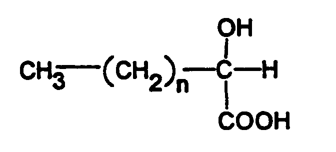
(c) ω-hydroxycarboxylic acids according to the formula
HO-CH₂-(CH₂)ₙ-CH₂-COOH
(d) isocarboxylic acids according to the formula 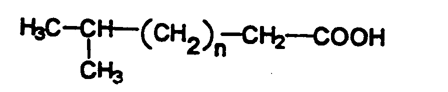
(e) α-hydroxy-isocarboxylic acids according to the formula 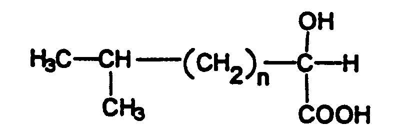
(f) ω-hydroxy-isocarboxylic acids according to the formula 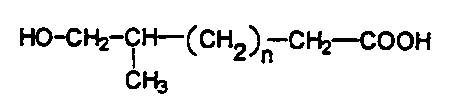
(g) anteisocarboxylic acids according to the formula 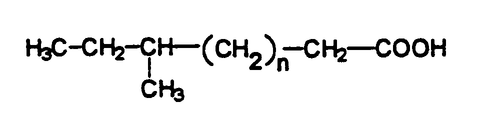
(h) α-hydroxy-anteisocarboxylic acids according to the formula 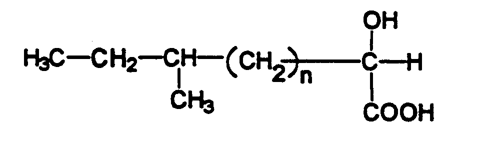
(i) ω-hydroxy-anteisocarboxylic acids according to the formula 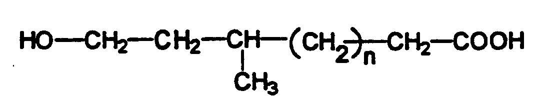
wherein n in each case assumes values of 7-31.

4. Cosmetic deodorants according to Claim 1, characterized in that wool wax acid mixtures in which the content of α-hydroxycarboxylic acids is at least 20-30 % by weight, based on the total composition, are used.

5. Cosmetic deodorants according to Claim 1, characterized in that the wool wax acids are distinguished by the following characterizing parameters:
| | |
|---|---|
| Dropping point | 50-54°C |
| Acid number | 166-170 |
| Saponification number | 175-190 |
| OH number | 60-80 |
| Iodine number | 7-12. |

6. Cosmetic deodorants according to Claim 1, characterized in that the wool wax acids or the wool wax acid components are present in concentrations of 0.05-10.00 % by weight, preferably 0.1-5.0 % by weight, and the α,ω-alkanedicarboxylic acid or the α,ω-alkanedicarboxylic acids are present in concentrations of 0.01-10.00 % by weight, preferably 0.05-5.0 % by weight, in each case based on the total weight of the formulations.

7. Cosmetic deodorants according to Claim 1, characterized in that the content of
I) α,ω-alkanedicarboxylic acids and
II) wool wax acids or wool wax acid components
is chosen such that ratios of I) and II) of 5:1 to 1:5, in particular about 1:1, result.

8. Cosmetic use of mixtures of
I) α,ω-alkanedicarboxylic acids and
II) wool wax acids or wool wax acid components,
which can be present, if appropriate, in a suitable cosmetic carrier, for combating Gram-positive, in particular coryneform bacteria, for combating the body odour caused by microbial decomposition of apocrine perspiration.

## Revendications

1. Déodorants cosmétiques contenants, à une concentration efficace, des mélanges
I) d'acides α,ω-alcanedicarboxyliques et
II) d'acides de lanoline ou de composants d'acides de lanoline.

2. Déodorants cosmétiques selon la revendication 1, caractérisés en ce que les acides α,ω-alcanedicarboxyliques selon l'invention sont choisis dans le groupe des substances qui sont décrites par la formule générique 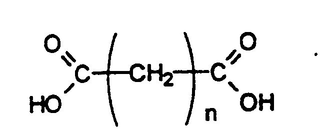 n représentant un noire allant de 1 à 8.

3. Déodorants cosmétiques selon la revendication 1, caractérisés en ce que les composants d'acides de lanoline sont choisis dans l'ensemble constitué par
(a) des acides carboxyliques saturés non substitués, correspondant à la formule C
H₃-(CH₂)ₙ-CH₂-COOH,
(b) des acides α-hydroxycarboxyliques, correspondant à la formule 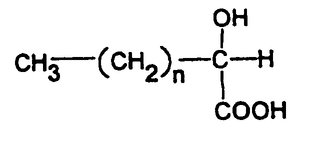
(c) des acides ω-hydroxycarboxyliques, correspondant à la formule
HO-CH₂-(CH₂)ₙ-CH₂-COOH
(d) des acides isocarboxyliques, correspondant à la formule 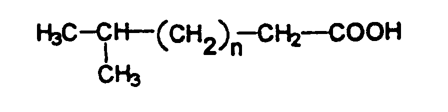
(e) des acides α-hydroxy-isocarboxyliques, correspondant à la formule 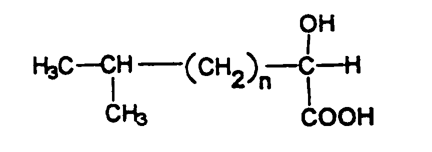
(f) des acides ω-hydroxy-isocarboxyliques, correspondant à la formule 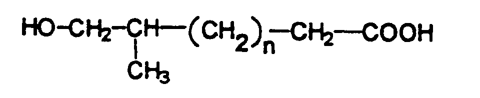
(g) des acides anté-isocarboxyliques, correspondant à la formule 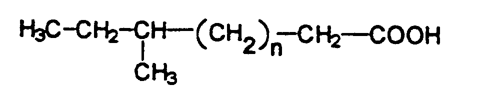
(h) des acides α-hydroxy-anté-isocarboxyliques, correspondant à la formule 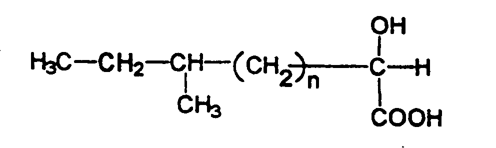
(i) des acides ω-hydroxy-anté-isocarboxyliques, correspondant à la formule 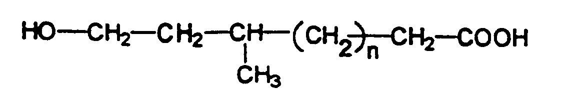
n valant dans chaque cas des valeurs de 7 à 31.

4. Déodorants cosmétiques selon la revendication 1, caractérisés en ce que l'on utilise des mélanges d'acides de lanoline dans lesquels la teneur en acides α-hydroxycarboxyliques est au moins de 20-30 % en poids, par rapport à la composition totale.

5. Déodorants cosmétiques selon la revendication 1, caractérisés en ce que les acides de lanoline se distinguent par les paramètres caractérisants suivants:
| | |
|---|---|
| Point de suintement | 50 - 54°C |
| Indice d'acide | 166 - 170 |
| Indice de saponification | 175 - 190 |
| Indice d'OH | 60 - 80 |
| Indice d'iode | 7 - 12. |

6. Déodorants cosmétiques selon la revendication 1, caractérisés en ce que les acides de lanoline ou les composants d'acides de lanoline sont présents à des concentrations de 0,05-10,00 % en poids, de préférence de 0,1-5,0 % en poids, et l'acide α,ω-alcanedi-carboxylique ou les acides α,ω -alcanedicarboxyliques sont présents à des concentrations de 0,01-10,00 % en poids, de préférence de 0,05-5,0 % en poids, dans chaque cas par rapport au poids total des compositions.

7. Déodorant cosmétique selon la revendication 1, caractérisé en ce que la teneur en
I) acides α,ω-alcanedicarboxyliques et
II) acides de lanoline ou composants d'acides de lanoline est choisie de manière qu'il en résulte des rapports de I) à II) tels que de 5:1 à 1:5, en particulier tels qu'environ 1:1.

8. Utilisation cosmétique de mélanges
I) d'acides α,ω-alcanedicarboxyliques et
II) d'acides de lanoline ou de composants d'acides de lanoline,
qui peuvent éventuellement se trouver dans un véhicule cosmétique approprié, pour la lutte contre des bactéries Gram positives, en particulier corynéformes, pour lutter contre l'odeur corporelle provoquée par la décomposition microbienne de la sueur apocrine.
